# EUROPÄISCHE PATENTSCHRIFT

(11) **EP 0 823 247 B1**
(45) Veröffentlichungstag und Bekanntmachung des Hinweises auf die Patenterteilung: **19.01.2000**
(21) Anmeldenummer: 97112347.6
(22) Anmeldetag: 18.07.1997
(51) Int. Cl.: A61F 9/007, A61B 17/02

(54) **Operationshaken für die Augenchirurgie**
Surgery hook for ophthalmic surgery
Crampon chirurgical pour chirurgie oculaire

(30) Priorität: 07.08.1996 CH 193496
(43) Veröffentlichungstag der Anmeldung: 11.02.1998
(73) Patentinhaber: GRIESHABER & CO. AG SCHAFFHAUSEN, CH-8203 Schaffhausen (CH)
(72) Erfinder: Grieshaber, Hans R., 8200 Schaffhausen (CH); Kazuaki, Kadonosono, Yokohama 236 (JP)
(74) Vertreter: Althoff, Gerhard

(56) Entgegenhaltungen:
- SOVIET PATENT ABSTRACTS Section PQ, Week 9229 Derwent Publications Ltd., London, GB; Class P32, AN 92-241227 XP002056952 & SU 1 676 619 A (EYE DISEASES RES INST)
- SOVIET PATENT ABSTRACTS Section PQ, Week 9423 Derwent Publications Ltd., London, GB; Class P32, AN 94-189808 XP002056953 & SU 1 803 081 A (EYE MICROSURGERY RES TECH INST)

## Beschreibung

Die Erfindung bezieht sich auf einen Operationshaken zur Verwendung beim Eingriff am Auge eines Lebewesens, mit einem länglichen Führungskörper, einem aus einem Bogenteil und einem Schenkel etwa hakenförmig zum Zurückziehen der Regenbogenhaut ausgebildeten Einhängeteil und einem in Längsrichtung an dem Führungskörper verschiebbar angeordneten Fixierelement, mittels welchem der mit dem Einhängeteil durch einen Einschnitt in die Vorderkammer eingeführte Führungskörper an der äusseren Kontur des Auges gehalten ist.

Bei der Augenchirurgie besteht das Problem, dass die Patienten auf medikamentöse Vergrösserung der Iris nicht ansprechen oder die Vergrösserung der Pupillenöffnung für den operativen Eingriff nicht ausreichend ist. Insbesondere bei Katarakt-Operationen ist es für den operativen Eingriff im vorderen Augenabschnitt aber auch bei Eingriffen im hinteren Augenabschnitt erforderlich ein ausreichend grosses, während des operativen Eingriffs unverändert bestehenbleibendes Sichtfeld für den Operateur zu gewährleisten. Zur Bildung des entsprechenden Sichtfeldes ist es bekannt, mittels einem oder mehrerer im Abstand zueinander angeordneter Operationshaken (Iris-Retractor) die Regenbogenhaut (Iris) zu erfassen und zur Vergrösserung der Pupillenöffnung nach aussen zu ziehen. Die einzelnen Operationshaken werden dabei jeweils durch einen entsprechend in der Hornhaut (Cornea) vorgesehenen Einschnitt in die Vorderkammer eingeführt und am Auge positioniert sowie nach dem Eingriff wieder entfernt.

Ein Operationshaken dieser Art ist aus EP-A 0 502 258 bekannt, welcher einen Führungskörper mit angeformtem und etwa hakenförmig ausgebildeten Einhängeteil sowie ein am Führungskörper verschiebbares, plättchenförmig ausgebildetes Klemmteil umfasst. Bei diesem Operationshaken ist das von dem Führungskörper durchdrungene Klemmteil mindestens auf der der äusseren Corneakontur zugewandten Seite mit einer kreisbogenförmigen Ausnehmung versehen, durch welche das Klemmteil etwa in zwei Teilstücke unterteilt und dadurch eine optimale Anpassung an die äussere Corneakontur gewährleistet ist.

Aus der EP-A 0 653 197 ist ein weiterer Operationshaken der erwähnten Art bekannt, welcher aus einem flexiblen, thermoplastischen Kunststoff hergestellt ist und im wesentlichen einen Führungskörper, ein daran in Längsrichtung verschiebbares Fixierelement sowie ein an dem einen Ende angeordnetes und bogenförmig am Führungskörper angeformtes Einhängeteil umfasst, welches durch eine Wärmebehandlung eine begrenzte Steifigkeit aufweist und beim Herausziehen des Führungskörpers aus dem Auge mindestens teilweise in eine etwa gestreckte Lage aufbiegbar ist.

Der Erfindung liegt die Aufgabe zugrunde, einen Operationshaken der bekannten und eingangs genannten Art dahingehend zu verbessern und so auszubilden, dass dieser zum Zurückziehen eines relativ grossen Bereichs der Regenbogenhaut (Iris) ohne Einreissen derselben sowie unter Beibehaltung einer präzisen Handhabung durch einen relativ kleinen, selbstabdichtenden Einschnitt in die Vorderkammer des Auges einführbar ist.

Diese Aufgabe wird erfindungsgemäss dadurch gelöst, dass der Führungskörper zwei in Längsrichtung parallel zueinander angeordnete sowie an den einander zugewandten Längskanten fest miteinander verbundene und jeweils an mindestens einem Ende mit einem Einhängeteil versehene Führungsarme aufweist, bei welchen die beiden korrespondierend zueinander angeordneten Einhängeteile ausgehend von den miteinander verbundenen Längskanten unter einem Winkel etwa Λ-förmig nach unten auseinanderlaufend zueinander angeordnet sind, derart, dass die beiden am Bogenteil angeordneten und etwa parallel zueinander verlaufenden Schenkel in Abhängigkeit des Winkels im Abstand zueinander angeordnet sind.

Weitere Merkmale der Erfindung ergeben sich aus der Beschreibung in Verbindung mit der Zeichnung und den weiteren Patentansprüchen.

Die Erfindung wird nachstehend anhand der Zeichnung beschrieben. Es zeigt:
**Fig.1** einen in Ansicht und in grösserem Massstab dargestellten und mit einem ersten und zweiten Führungsarm sowie mit jeweils daran angeordnetem Einhängeteil versehenen Führungskörper für einen Operationshaken;
**Fig.2** den in räumlicher Ansicht dargestellten Führungskörper mit den beiden parallel zueinander angeordneten sowie miteinander verbundenen Führungsarmen;
**Fig.3** den in räumlicher Draufsicht dargestellten Führungskörper mit den beiden miteinander verbundenen Führungsarmen;
**Fig.4** den gemäss Pfeilrichtung IV in Fig.2 etwa in räumlicher Unteransicht dargestellten Führungskörper;
**Fig.5** den in Ansicht dargestellten Operationshaken mit dem Führungskörper sowie einem daran angeordneten Fixierelement;
**Fig.6** den in Draufsicht dargestellten und mit den einzelnen Elementen versehenen Operationshaken gemäss Fig.5;
**Fig.7** die gemäss Pfeilrichtung VII in Fig.2 in Seitenansicht dargestellte Anordnung der beiden Einhängeteile des Führungskörpers;
**Fig.8** ein schematisch und als Horizontalschnitt vergrössert dargestelltes Teilstück eines Auges, bei welchem ein Bereich der Regenbogenhaut (Iris) mittels des Operationshakens zurückgezogen dargestellt ist;
**Fig.9** das in Draufsicht und in bezug auf Fig.8 kleiner dargestellte Auge mit teilweise zurückgezogener Regenbogenhaut (Iris); und
**Fig.10** das vordere Teilstück eines der beiden Einhängeteile gemäss Fig.1, bei welchem das eine hakenförmige Bogenteil in weitgehend geöffnetem beziehungsweise gestrecktem Zustand dargestellt ist.

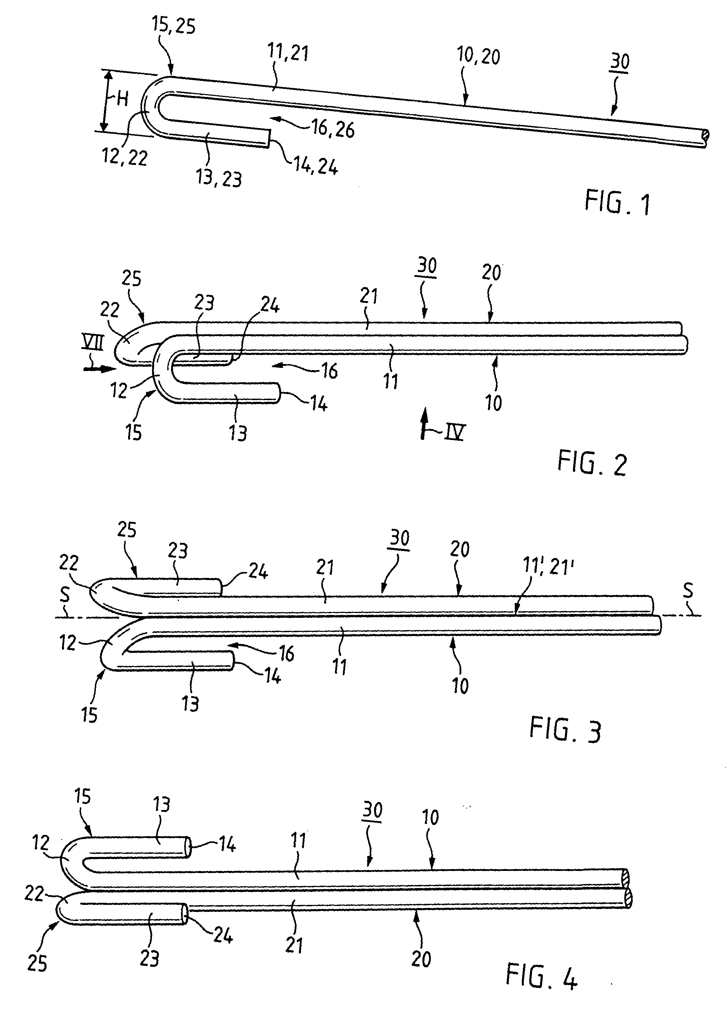

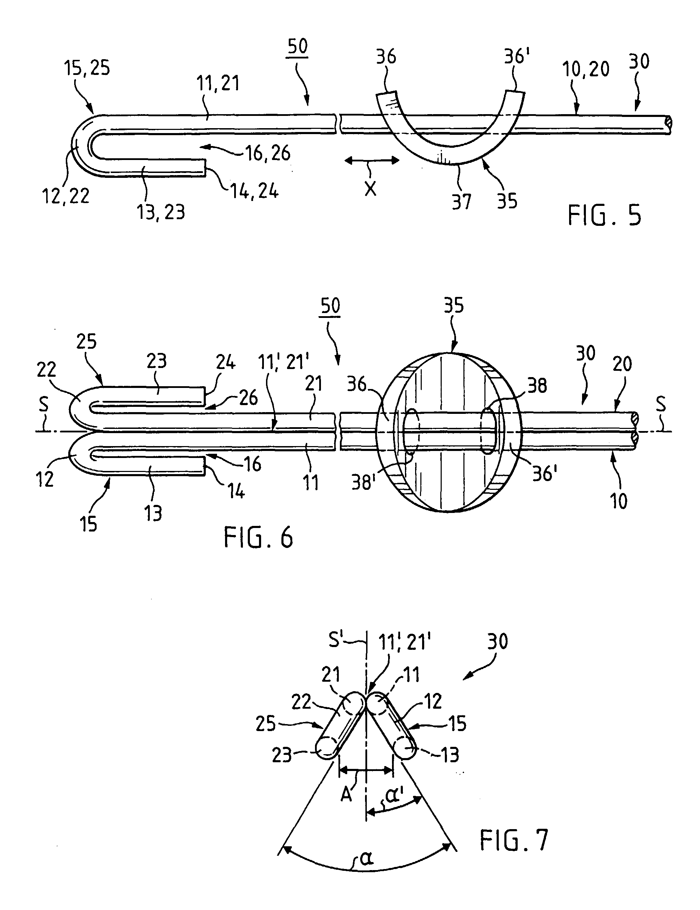

Fig.1 zeigt einen in Ansicht sowie in grösserem Massstab dargestellten Führungskörper 30 für einen Operationshaken, mittels welchem für einen operativen Eingriff am Auge eines Lebewesens die Regenbogenhaut (Iris) erfasst und etwa in Richtung des Übergangs von der Hornhaut zur Lederhaut nach aussen gezogen wird. Der Führungskörper 30 umfasst zwei längliche und parallel zueinander angeordnete Führungsarme 10 und 20, welche jeweils an dem einen Ende mit einem Einhängeteil 15 und 25 versehen sind. Die einzelnen Teile sind in der äusseren Formgebung gleich ausgebildet und mit nicht dargestellten Mitteln miteinander verbunden. Die spezielle Anordnung der beiden Führungsarme 10 und 20 und der Einhängeteile 15 und 25 zueinander ist jeweils in Figur 2 bis 4 sowie in Figur 7 dargestellt.

Die beiden analog ausgebildeten Führungsarme 10 und 20 sind aus einem Faden mit absolut glatter Oberfläche hergestellt und haben jeweils ein längliches Führungsstück 11 und 21 und mindestens an dem einen Ende das hakenförmig ausgebildete Einhängeteil 15 und 25. Das etwa halbkreisbogenförmig ausgebildete Einhängeteil 15,25 ist jeweils an dem einen Ende des Führungsstücks 11,21 angeformt und umfasst ein Bogenteil 12 und 22 und einen durch einen Spalt 16 und 26 im Abstand zum Führungsstück 11,21 angeordneten Schenkel 13 und 23. Die Enden der beiden Schenkel 13,23 sind jeweils mit einer rechtwinkelig dazu orientierten Stirnkante 14 und 24 oder aber mit einer nicht dargestellten, abgerundet ausgebildeten Stirnkante versehen. Das mit dem Bogenteil 12,22 einmal haarnadelförmig umgebogene und an dem Führungsstück 11,21 angeformte Einhängeteil 15,25 ist derart ausgebildet, dass der Schenkel 13,23 etwa parallel und durch den Spalt 16,26 im Abstand zu dem jeweiligen Führungsstück 11,21 angeordnet ist.

Fig.2 zeigt den etwa in räumlicher Ansicht dargestellten Führungskörper 30 und man erkennt die beiden parallel zueinander angeordneten sowie miteinander verbundenen Führungsarme 10 und 20 mit dem daran angeformten Einhängeteil 15 und 25. Die beiden länglichen Führungsarme 10 und 20 sind mittels der beiden Führungsstücke 11 und 21 derart zueinander angeordnet und in nicht näher dargestellter Weise fest miteinander verbunden, dass die beiden am Bogenteil 12,22 angeformten Schenkel 13,23 der Einhängeteile 15,25, wie bereits erwähnt, jeweils durch den Spalt 16,26 im Abstand zum Führungsstück 11,21 angeordnet sind.

In Fig.3 ist der Führungskörper 30 in räumlicher Draufsicht und in Fig.4 gemäss Pfeilrichtung IV in Fig.2 in räumlicher Unteransicht dargestellt und man erkennt die beiden entlang einer theoretischen Symmetrieachse S-S (Fig.3) bis etwa in den vorderen Bereich aneinander liegenden und miteinander befestigten Führungsarme 10 und 20 sowie die im vorderen Bereich an den beiden Führungsstücken 11 und 21 angeformten und relativ zueinander nach unten auseinanderlaufend ausgebildeten Einhängeteile 15,25 mit den Bogenteilen 12,22 und Schenkeln 13,23.

Fig.5 zeigt den in Ansicht und Fig.6 den in Draufsicht dargestellten und in der Gesamtheit mit 50 bezeichneten Operationshaken und man erkennt den die beiden Einhängeteile 15,25 und Führungsarme 10,20 umfassenden Führungskörper 30 sowie ein daran angeordnetes Fixierelement 35. Das beispielsweise als kreisförmige Scheibe ausgebildete und aus flexiblem Material hergestellte Fixierelement 35 hat, wie in Fig.6 dargestellt, zwei im Abstand zueinander angeordnete und das Fixierelement 35 durchdringende Ausnehmungen 38 und 38'. Die beiden Ausnehmungen 38,38' sind derart ausgebildet und zueinander angeordnet, dass das Fixierelement 35 auf den beiden miteinander verbundenen Führungsarmen 10,20 des Führungskörpers 30 in Richtung des Doppelpfeils X (Fig.5) verschoben werden kann. Hierbei wird das Fixierelement 35 beispielsweise derart verformt, dass auf der einen Seite des Führungskörpers 30 eine etwa bogenförmige Anlage 37 gebildet wird, während auf der anderen Seite des Führungskörpers 30 zwei im Abstand zueinander angeordnete Bereiche 36,36' des Fixierelements 35 hervorstehen (Fig.5).

Zum Verschieben des Fixierelements 35 auf den beiden Führungsarmen 10,20 des Führungskörpers 30 werden die beiden Bereiche 36,36' relativ zueinander zusammengedrückt, wodurch die nicht bezeichneten Innenkanten der Ausnehmungen 38,38' etwa ausser Eingriff der Führungsarme 10,20 gelangen (nicht dargestellt). Durch Loslassen der beiden Bereiche 36,36' wird das Fixierelement 35 in beliebiger Position auf dem Führungskörper 30 selbsthemmend gehalten.

In Fig.6 ist der Operationshaken 50 in Draufsicht dargestellt und man erkennt den Führungskörper 30 mit den beiden Führungsarmen 10 und 20, das daran angeordnete Fixierelement 35 sowie die beiden parallel zueinander angeordneten Führungsteile 11,21 mit den jeweils am vorderen Ende daran angeformten Teilen 12,13,15 und 22,23,25. Die beiden beabstandeten und auf der gemeinsamen Symmetrieachse S-S des Führungskörpers 30 sowie des Fixierelements 35 angeordneten Bohrungen 38 und 38' sind vorzugsweise so ausgebildet und derart dimensioniert, dass das Fixierelement 35 selbsthemmend auf den beiden Führungsarme 10 und 20 angeordnet und durch Zusammendrücken der beiden Bereiche 36 und 36' in Richtung des Doppelpfeils X (Fig.5) in axialer Richtung an dem Führungskörper 30 verstellbar ist. Die Ausnehmungen 38,38' in dem scheibenförmigen Fixierelement 35 sind etwa analog dem jeweils kreisförmigen Profilquerschnitt der beiden aneinanderliegend und fest miteinander verbundenen Führungsarme 10 und 20 ausgebildet.

Fig.7 zeigt gemäss Pfeilrichtung VII in Fig.2 die in Seitenansicht dargestellte Anordnung und die auf eine vertikale Achse S' bezogene Relativlage der beiden Führungsarme 10 und 20 zueinander. Die in Längsrichtung orientierten Führungsteile 11 und 21 der Führungsarme 10,20 sind mit den einander zugewandten Längskanten 11',21' durch nicht dargestellte Mittel fest miteinander verbunden. Ausgehend von den aneinanderliegenden Längskanten 11',21' sind die beiden Einhängeteile 15 und 25 unter spitzem Winkel α etwa Λ-förmig nach unten auseinanderlaufend zueinander angeordnet, wobei die beiden Schenkel 13 und 23 durch einen Abstand A parallel zueinander angeordnet sind. Die beiden jeweils am Bogenteil 12,22 angeordneten und parallel zueinander nach hinten verlaufenden Schenkel 13,23 sind in Abhängigkeit des Winkels α durch den Abstand A zueinander angeordnet.

Die beiden Führungsarme 10 und 20 sind um ihre Längsachse derart verdreht und miteinander fest verbunden, dass der spitze Winkel α zwischen den beiden Einhängeteilen 15 und 25 beispielsweise in der Grössenordnung von 30° bis 60° liegt. Bei einer nicht dargestellten Ausführungsvariante besteht jedoch auch die Möglichkeit, dass das eine Einhängeteil 15 oder das andere Einhängeteil 25 auf der vertikalen Achse S' angeordnet und das in Abhängigkeit davon gegenüberliegende Einhängeteil unter einem Winkel α' relativ dazu angeordnet ist.

An dieser Stelle wird darauf hingewiesen, dass zur Herstellung der beiden Führungsarme 10 und 20 jeweils ein verhältnismässig flexibler Faden aus thermoplastischem Kunststoff, beispielsweise ein Polyamidfaden mit guten Oberflächengleiteigenschaften verwendet wird. Zur Erreichung der in Fig.1 dargestellten Formgebung des einzelnen Führungsarms 10 und 20 mit den angeformten Teilen 12,13 und 22,23 wird der Faden durch eine Wärmebehandlung verformt.

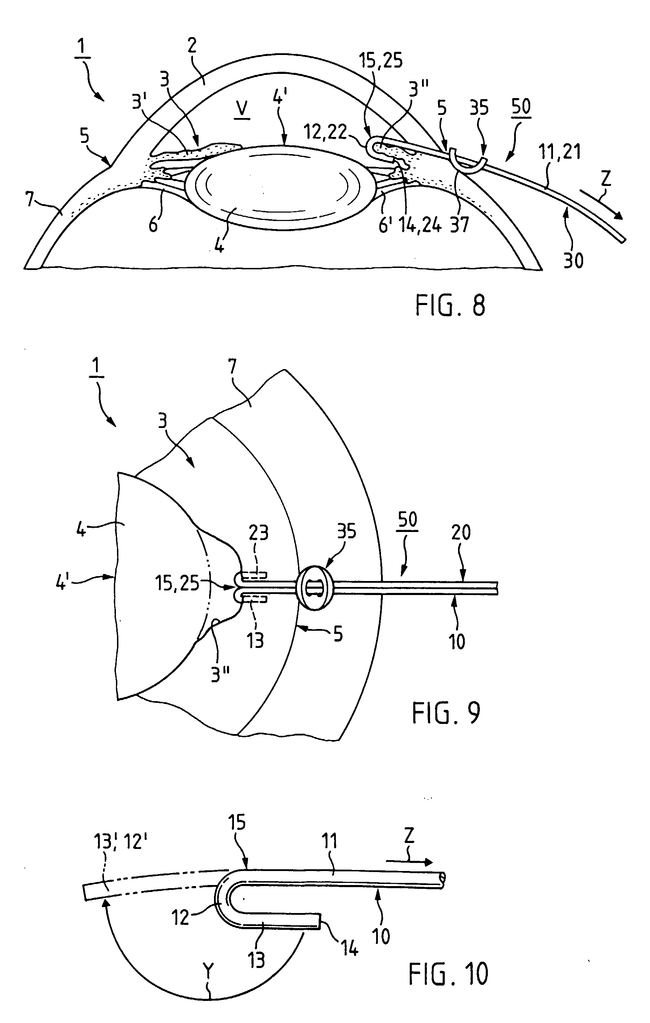

Für die entsprechende Formgebung der Führungsarme 10,20 wird der Polyamidfaden beispielsweise auf einem dem Führungsteil 11,21 sowie dem Einhängeteil 15,25 entsprechend ausgebildeten Formkörper (nicht dargestellt) aufgebracht und zusammen mit diesem einer Wärmebehandlung (Temperung) unterzogen. Durch die Wärmebehandlung erhält der Polyamidfaden im Bereich des Einhängeteils 15,25 die hakenförmige Formgebung und dadurch eine lokale Steifigkeit. Das in bezug auf das Einhängeteil 15 und 25 relativ lange Führungsteil 11 und 21 bleibt nach der Wärmebehandlung weitgehend flexibel. Das einzelne Führungsteil 11,21 kann dabei gerade oder aber, wie in Fig.8 schematische dargestellt mit relativ grossem Radius etwa bogenförmig ausgebildet werden. Anschliessend werden die beiden Führungsteile 11,21 durch nicht näher dargestellte Mittel, beispielsweise durch eine geeignete Klebverbindung (nicht dargestellt) über die gesamte Länge, wie in Fig.7 dargestellt, unter dem Winkel α fest zueinander angeordnet miteinander verbunden.

Das Fixierelement 35 ist beispielsweise aus flexiblem Kunststoff mit guten Gleiteigenschaften hergestellt, welches beim Zusammendrücken der beiden Bereiche 36,36' weitgehend kraftlos auf den Führungsteilen 11,21 des Führungskörpers 30 verschiebbar ist. Das Fixierelement 35 ist vorzugsweise aus transparentem Silikon (Silikon-Kautschuk) hergestellt.

Fig.8 zeigt ein in der Gesamtheit mit 1 bezeichnetes Auge in schematisch dargestelltem Horizontalschnitt und man erkennt die Hornhaut 2 (Cornea), die Regenbogenhaut 3 (Iris) mit den beiden äusseren Bereichen 3' und 3", die Lederhaut 7 (Sklera), die Linse 4 (Okular), die Pupille 4', die Strahlenbänder 6,6' (Zonula) sowie die in der Gesamtheit mit V bezeichnete Vorderkammer.

Zur Verdeutlichung der Erfindung ist in Fig.8 der mit den beiden Einhängeteilen 15 und 25 an dem einen Bereich 3" der Iris 3 eingehängte Operationshaken 50 dargestellt. Der Operationshaken 50 ist mittels der weitgehend am Übergang 5 anliegenden und etwa bogenförmig geformten Anlage 37 des Fixierelements 35 gehalten. Die flexibel und leicht bogenförmig ausgebildeten Führungsarme 10,20 des Führungskörpers 30 sind dabei etwa an die äussere Kontur der Lederhaut 7 (Sklera) angepasst. Hierdurch wird erreicht, dass auch bei mehreren, zirkulär im Abstand zueinander verteilt angeordneten Operationshaken 50 ein gut zugänglicher Operationsbereich gewährleistet ist und der Operateur beim Eingriff nicht an dem abstehenden Führungskörper 30 des einzelnen Operationshakens 50 hängen bleibt.

Eine unbeabsichtigte Berührung des Führungskörpers 30 durch den Operateur wird durch die Flexibilität der länglichen Führungsarme 10,20 weitgehend kompensiert, so dass bei unbeabsichtigter Berührung keine gefährliche Bewegung (Herausreissen des Operationshakens 50 oder Einwirkung auf die Linse 4) auf das Auge 1 übertragen werden kann.

Fig.9 zeigt ein in Draufsicht dargestelltes Teilstück des Auges 1 und man erkennt das mittels der beiden Einhängeteile 15,25 des Operationshakens 50 zurückgezogene Teilstück 3" der Iris 3. Der Operationshaken 50 ist dabei mit dem scheibenförmigen Fixierelement 35 am Übergang 5 von der Lederhaut 7 zu der hier nicht dargestellten Hornhaut 2 des Auges 1 gehalten.

In Fig.10 ist als Ausführungsbeispiel das Einhängeteil 15 des einen Führungsarms 10 dargestellt, wobei das Einhängeteil 25 des anderen Führungsarms 20 analog ausgebildet ist. Durch die erwähnte Wärmebehandlung des Polyamidfadens wird im Bereich der Einhängeteile 15 und 25 eine lokale Steifigkeit erreicht, so dass sich die beiden Bogenstücke 12 und 22 entsprechend aufbiegen lassen. Die an den beiden Führungsteilen 11,21 des Führungskörpers 30 angeformten Einhängeteile 15 und 25 sind, wie vorstehend erwähnt, durch die spezielle Wärmebehandlung derart verformt, dass die Bogenteile 12 und 22 entgegen der federelastischen Rückstellkraft, wie in Fig.10 schematisch dargestellt, gemäss Pfeilrichtung Y in die etwa gestreckte Lage der Teile 13',12' aufbiegbar und anschliessend weitgehend wieder in die durch die Wärmebehandlung erreichte Formgebung zurückführbar sind. Durch diese Massnahme ist der komplette Operationshaken 50 in vorteilhafter Weise mehrfach verwendbar.

Zur Verdeutlichung der Dimensionen des erfindungsgemässen Operationshakens 50 wird darauf hingewiesen, dass die gesamte Länge des Führungskörpers 30 mit den beiden Einhängeteilen 15,25 jeweils etwa in der Grössenordnung zwischen 5 mm bis 8 mm und die Länge der haarnadelförmig umgebogenen Schenkel 13,23 jeweils in der Grössenordnung zwischen 1,0 mm bis 1,5 mm liegen. Die gesamte Höhe H (Fig.1) des einzelnen Bogenteils 12,22 liegt etwa in der Grössenordnung zwischen 0,4 mm bis 0,5 mm. Der Polyamidfaden für den einzelnen Führungsarm 10 und 20 ist beispielsweise im Profilquerschnitt kreisförmig ausgebildet und hat einen Durchmesser von etwa 0,15 mm bis 0,2 mm.

Zum Entfernen des eingesetzten Operationshakens 50 wird dieser nach dem Zurückziehen des Fixierelements 35 beispielsweise entgegen der Pfeilrichtung Z (Fig.8) in nicht näher dargestellter Weise relativ zu dem Teilstück 3" der Iris 3 in die Vorderkammer V geschoben. Sobald die beiden Einhängeteile 15,25 ausser Eingriff des Teilstücks 3" sind wird der Führungskörper 30 mit einem Winkel von etwa 70° bis 90° um seine Längs- und Symmetrieachse S-S gedreht (nicht dargestellt). Anschliessend kann der Führungskörper 30 in Pfeilrichtung Z herausgezogen werden. Sobald der Operationshaken 50 vollständig herausgezogen und entfernt ist, erfolgt ein selbstabdichtendes Verschliessen des in der Hornhaut 2 vorgesehenen und nicht näher dargestellten Einschnittes.

Zum Entfernen des eingesetzten Operationshakens 50 besteht jedoch auch die Möglichkeit, dass ausgehend von der in Fig.8 dargestellten Stellung die beiden Einhängeteile 15,25 wie vorstehend beschrieben ausser Eingriff des Teilstücks 3" der Iris 3 gebracht und anschliessend in Pfeilrichtung Z gezogen werden. Sobald die beiden Stirnseiten 14,24 der Schenkel 13,23 den nicht bezeichneten Übergang von der Hornhaut 2 (Cornea) zur Lederhaut 7 (Sklera) kontaktieren, werden die beiden Einhängeteile 15,25 durch den natürlichen Widerstand des Übergangs entsprechend aufgebogen, so dass der Führungskörper 30 in Pfeilrichtung Z problemlos herausgezogen werden kann, ohne dass dabei der Einschnitt in der Hornhaut 2 (Cornea) durch Aufreissen unerwünscht vergrössert wird. Auch bei dieser Variante erfolgt anschliessend ein selbstabdichtendes Verschliessen des in der Hornhaut 2 vorgesehenen Einschnittes.

## Patentansprüche

1. Operationshaken zur Verwendung beim Eingriff am Auge eines Lebewesens, mit einem länglichen Führungskörper (30), einem aus einem Bogenteil (12, 22) und einem Schenkel (13, 23) etwa hakenförmig zum Zurückziehen der Regenbogenhaut ausgebildeten Einhängeteil (15, 25) und einem in Längsrichtung an dem Führungskörper verschiebbar angeordneten Fixierelement (35), mittels welchem der mit dem Einhängeteil durch einen Einschnitt in die Vorderkammer eingeführte Führungskörper an der äusseren Kontur des Auges gehalten ist, **dadurch gekennzeichnet,** dass der Führungskörper (30) zwei in Längsrichtung parallel zueinander angeordnete sowie an den einander zugewandten Längskanten (11',21') fest miteinander verbundene und jeweils an mindestens einem Ende mit einem Einhängeteil (15,25) versehene Führungsarme (10,20) aufweist, bei welchen die beiden Einhängeteile (15,25) ausgehend von den miteinander verbundenen Längskanten unter einem Winkel (α) etwa Λ-förmig nach unten auseinanderlaufend zueinander angeordnet sind, derart, dass die beiden am Bogenteil (12,22) angeordneten und etwa parallel zueinander verlaufenden Schenkel (13,23) in Abhängigkeit des Winkels (α) mit einem Spalt (16,26) im Abstand zueinander angeordnet sind.

2. Operationshaken nach Anspruch 1, **dadurch gekennzeichnet,** dass die beiden länglichen Führungsarme (10,20) relativ zueinander derart um die eigene Längsachse verdreht und fest miteinander verbunden sind, dass die am vorderen Ende angeordneten Einhängeteile (15,25) unter spitzem Winkel (α) im Abstand (A) zueinander angeordnet sind.

3. Operationshaken nach den Ansprüchen 1 und 2, **dadurch gekennzeichnet,** dass die an den Führungsarmen (10,20) angeformten Einhängeteile (15,25) unter spitzem Winkel (α) in der Grössenordnung von etwa 30° bis 60° im Abstand (A) zueinander angeordnet sind.

4. Operationshaken nach Anspruch 1, **dadurch gekennzeichnet,** dass die beiden länglichen Führungsarme (10,20) derart miteinander befestigt sind, dass das eine Einhängeteil auf der quer zur Symmetrieachse (S-S) orientierten vertikalen Achse (S') angeordnet und das gegenüberliegende andere Einhängeteil unter spitzem Winkel (α**'**) im Abstand dazu angeordnet ist.

5. Operationshaken nach Anspruch 1, **dadurch gekennzeichnet,** dass die beiden Führungsarme (10;20) des Führungskörpers (30) im Profilquerschnitt kreisförmig ausgebildet und mit den einander zugewandten Längskanten (11',21') der Führungsteile (11,21) eng aneinanderliegend durch geeignete Mittel, beispielsweise in Form einer in Längsrichtung orientierten Klebverbindung, fest miteinander verbunden sind.

6. Operationshaken nach den Ansprüchen 1 bis 5, **dadurch gekennzeichnet,** dass der einzelne Führungsarm (10,20) aus einem flexiblen thermoplastischen Kunststofffaden, insbesondere aus einem Polyamidfaden hergestellt ist, bei welchem das hakenförmig ausgebildete Einhängeteil (15,25) jeweils durch eine thermische Behandlung angeformt ist.

7. Operationshaken nach Anspruch 6, **dadurch gekennzeichnet,** dass die thermisch an den Führungsteilen (11,21) angeformten Einhängeteile (15,25) jeweils eine begrenzte, lokale Steifigkeit aufweisen und infolge eines verhältnismässig geringen Widerstandes in eine weitgehend gerade Stellung aufbiegbar und anschliessend durch die eigene federelastische Rückstellkraft selbsttätig in die hakenförmige Formgebung zurückführbar sind.

8. Operationshaken nach Anspruch 1, **dadurch gekennzeichnet,** dass das Fixierelement (35) zwei gegenüberliegend im Abstand zueinander angeordnete Ausnehmungen (38,38') aufweist und gegen Verdrehung gesichert in Längsrichtung an den Führungsarmen (10,20) des Führungskörpers (30) verschiebbar geführt ist.

9. Operationshaken nach Anspruch 8, **dadurch gekennzeichnet,** dass das Fixierelement (35) scheibenförmig ist und die beiden Ausnehmungen (38,38') in dem scheibenförmigen Fixierelement (35) etwa analog dem Profilquerschnitt der beiden aneinanderliegend sowie fest miteinander verbundenen und im Profilquerschnitt jeweils kreisförmig ausgebildeten Führungsarme (10,20) ausgebildet sind.

10. Operationshaken nach den Ansprüchen 8 und 9, **dadurch gekennzeichnet,** dass aus transparentem Silikon-Kautschuk oder dergleichen hergestellt ist.

## Claims

1. A surgery hook for use in operating on the eye of a living being, comprising an elongated guide (30), a hook-in member (15, 25) formed by an arcuate portion (12, 22) and a limb (13, 23) substantially in a hook shape for drawing back the iris, and a fixing element (35) which may be moved longitudinally on the guide and by means of which the guide inserted in the anterior chamber by the hook-in member through an incision is held against the external contour of the eye, **characterised in that** the guide (30) has two guide arms (10, 20) which are parallel in the longitudinal direction, fixed together at the facing longitudinal edges (11', 21') and each provided with a hook-in member (15, 25) at at least one end, in which arms the two hook-in members (15, 25), starting from the interconnected longitudinal edges, diverge downwardly substantially in a Λ shape at an angle *α,* in such a way that the two limbs (13, 23) arranged on the arcuate portion (12, 22) and extending substantially parallel with each other are spaced from each other with a gap (16, 26) dependent on the angle α.

2. A surgery hook according to claim 1, **characterised in that** the two elongated guide arms (10, 20) are twisted around their own longitudinal axis relative to each other and fixed together, in such a way that the hook-in members (15, 25) at the front end arc arranged at a spacing (A) from each other at an acute angle α

3. A surgery hook according to claims 1 and 2, **characterised in that** the hook-in members (15, 25) integral with the guide arms (10, 20) are arranged at a spacing (A) from each other at an acute angle (α) in the order of approximately 30 to 60°.

4. A surgery hook according to claim 1, **characterised in that** the two elongated guide arms (10, 20) are fixed together in such a way that one hook-in member is located on the vertical axis directed transversely to the axis of symmetry (S-S) and the other, opposing hook-in member is spaced therefrom at an acute angle (α').

5. A surgery hook according to claim 1, **characterised in that** the two guide arms (10, 20) of the guide (30) are circular in the cross-section of their profile and, with the interfacing longitudinal edges (11', 21') of the guide pieces (11, 21) lying close together, are fixed together by suitable means, for example in the form of a longitudinally directed adhesive connection.

6. A surgery hook according to claims 1 to 5, **characterised in that** the individual guide arm (10, 20) is made from a flexible thermoplastic plastic thread, particularly a polyamide, in which the hook-shaped hook-in member (15, 25) is in each case moulded on by a heat treatment.

7. A surgery hook according to claim 6, **characterised in that** the hook-in members (15,25) moulded thermally onto the guide pieces (11,21) each have limited local stiffness and, as a result of a comparatively low resistance, can be bent open into a substantially straight position and then automatically brought back to the hooked shape by their own resilient return force.

8. A surgery hook according to claim 1, **characterised in that** the fixing element (35) has two apertures (38, 38') opposite and spaced from each other, and is displaceable along the guide arms (10, 20) of the guide (30) with protection from twisting.

9. A surgery hook according to claim 8, **characterised in that** the two apertures (38, 38') in the disc-shaped fixing element (35) are shaped substantially similarly to the cross-section of the profile of the two guide arms (10, 20), which lie against one another, are fixed together and are each circular in the cross-section of their profile.

10. A surgery hook according to claims 8 and 9, **characterised in that** the fixing element (35) is disc-shaped and made of transparent silicone rubber or the like.

## Revendications

1. Crochet chirurgical à utiliser lors d'une intervention sur l'oeil d'un être vivant, présentant un corps de guidage longitudinal (30), un élément d'accrochage (15,25) formé d'un élément coudé (12,22) et d'une branche (13,23) approximativement en forme de crochet et permettant de rétracter l'iris, et un élément de fixation (35) disposé de façon mobile par rapport à l'élément de guidage dans la direction longitudinale, au moyen duquel le corps de guidage introduit avec l'élément d'accrochage par une incision pratiquée dans la chambre antérieure est maintenu contre le contour externe de l'oeil, **caractérisé en ce que** le corps de guidage (30) présente deux bras de guidage (10,20) disposés parallèlement l'un à l'autre dans la direction longitudinale, reliés fixement l'un à l'autre au niveau des arêtes longitudinales (11',21') dirigées l'une vers l'autre et pourvues respectivement à au moins une extrémité d'un élément d'accrochage (15,25), dans lequel les deux éléments d'accrochage (15,25) en partant des arêtes longitudinales reliées l'une à l'autre, sont disposés en s'écartant vers le bas selon un angle (α) approximativement en forme de 1, de façon telle que les deux branches (13,23) disposées contre l'élément coudé (12,22) et s'étendant environ parallèlement l'une à l'autre sont disposées l'une par rapport à l'autre en formant un écart (16,26) qui est fonction de l'angle (α).

2. Crochet chirurgical selon la revendication 1, **caractérisé en ce que** les deux bras de guidage longitudinaux (10,20) pivotent l'un par rapport à l'autre autour de leur propre axe longitudinal et sont reliés fixement l'un à l'autre de façon telle que les éléments d'accrochage (15,25) disposés à l'extrémité avant sont disposés l'un par rapport à l'autre selon un angle aigu (α) en formant un écart (A).

3. Crochet chirurgical selon les revendications 1 et 2, **caractérisé en ce que** les éléments d'accrochage (15,25) façonnés sur les bras de guidage (10,20) sont disposés l'un par rapport à l'autre selon un angle aigu (α) de l'ordre de grandeur compris entre environ 30° et 60° pour former un écart (A).

4. Crochet chirurgical selon la revendication 1, **caractérisé en ce que** les deux bras de guidage longitudinaux (10,20) sont fixés l'un à l'autre de façon telle qu'un élément d'accrochage est disposé sur l'axe vertical (S') orienté perpendiculairement à l'axe de symétrie (S-S) et en ce que l'autre élément d'accrochage opposé est disposé selon un angle aigu (α') en formant un écart.

5. Crochet chirurgical selon la revendication 1, **caractérisé en ce que** les deux bras de guidage (10,20) du corps de guidage (30) sont formés avec une section circulaire et fermement reliés entre eux par les arêtes longitudinales (11',21') dirigées l'une vers l'autre des éléments de guidage (11,21) par des moyens appropriés, par exemple sous la forme d'une liaison collée orientée dans la direction longitudinale.

6. Crochet chirurgical selon les revendications 1 à 5, **caractérisé en ce que** le bras de guidage individuel (10,20) est fabriqué à partir d'un matériau thermoplastique flexible, particulièrement à partir d'un fil de polyamide, dans lequel l'élément d'accrochage (15,25) conçu en forme de crochet est respectivement formé par un traitement thermique.

7. Crochet chirurgical selon la revendication 6, **caractérisé en ce que** les éléments d'accrochage (15,25) façonnés thermiquement contre les éléments de guidage (11,21) présentent respectivement une rigidité locale limitée et peuvent, du fait d'une résistance relativement faible, être dépliés dans une position essentiellement droite puis reprendre automatiquement leur forme de crochet par leur propre force de retrait élastique.

8. Crochet chirurgical selon la revendication 1, **caractérisé en ce que** l'élément de fixation (35) présente deux évidements (38,38') disposés de façon opposée en formant un écart et peut être poussé, de façon protégée contre toute rotation, dans la direction longitudinale sur les bras de guidage (10,20) du corps de guidage (30).

9. Crochet chirurgical selon la revendication 8, **caractérisé en ce que** l'élément de fixation (35) est en forme de disque et les deux évidements (38,38') de l'élément de fixation (35) en forme de disque sont conçus de façon approximativement analogue à la coupe de profil des deux bras de guidage (10,20) placés l'un contre l'autre, reliés fermement l'un à l'autre et conçus respectivement en forme de cercle selon une coupe de profil circulaire.

10. Crochet chirurgical selon les revendications 8 et 9, **caractérisé en ce que** l'élément de fixation (35) est en forme de disque et est fabriqué à partir de caoutchouc siliconé transparent ou équivalent.
